# EUROPEAN PATENT SPECIFICATION

(11) **EP 3 689 169 B1**
(45) Date of publication and mention of the grant of the patent: **09.03.2022**
(21) Application number: 20153700.8
(22) Date of filing: 24.01.2020
(51) Int. Cl.: A42B 1/046, A42B 3/04, A42B 3/10

(54) **SENSORIZED GARMENT**
KLEIDUNGSSTÜCK MIT SENSOREN
VÊTEMENT AVEC CAPTEURS

(30) Priority: 31.01.2019 IT 201900001403
(43) Date of publication of application: 05.08.2020
(73) Proprietor: Comftech S.r.L., 20900 Monza (MB) (IT)
(72) Inventor: Moltani, Lara Alessia Laura, 20900 20900 Monza MB (IT); Orlandi, Luca Domenico, 20900 20900 Monza MB (IT)
(74) Representative: Perani & Partners S.p.A.

(56) References cited:
- US-A1- 2011 219 852
- US-A1- 2013 303 946
- US-B1- 9 775 396

## Description

### Field of the invention

The present invention relates to a sensorized garment having an under helmet hood for monitoring impacts and deformations occurring by contact with the body surface of a wearer.

In particular, the sensorized garment is configured to detect any impact suffered by the wearer, in particular, at the head of the wearer.

### Discussion of the related art

Sensorized garments are known to be provided for detection of the physiological electric signals and vital parameters of an individual.

This sensorized garment comprises suitable textile sensors that can detect the heart rate of the wearer. The heart rate signal associated is stored in a special associated database for future reference.

Prior art sensorized garments are widely used for both medical applications and sports, to monitor the vital parameters of an individual.

A sensor suit is known for sports such as motorcycle racing, which has acceleration sensors and a processing unit that processes the signals generated by the sensors and is thus able to recognize a fall or an imminent impact to immediately deploy the airbags within the suit.

A known sensorized garment is described in document US 2013/303946 A1, having an under helmet hood 2 made of a. textile material, which can be worn to cover the head of an individual.

### Problem of the prior art

As mentioned above, prior art suits, especially those used in sports applications, afford monitoring of the vital parameters of the individual and actuation of safety systems such as airbags. This is also the case for sports with vehicles such as bicycles, motorcycles, or generally requiring the use of a helmet and protective equipment to limit impacts.

Nevertheless, after an accident, the medical staff that care for the individual have to reconstruct the dynamics of the accident based on the information given by the individual concerned and other witnesses, as well as the overall scene of the accident. Such information may be unreliable as provided by people affected by trauma, even when they simply witness the accident. Furthermore, the reconstruction of the dynamics of the impact only based on the scene of the accident may be inaccurate for its being only theoretical deduced.

### SUMMARY OF INVENTION

The object of the present invention is to provide a sensorized garment that can obviate the drawbacks of the prior art.

A further object of the present invention is to provide a sensorized garment that can provide support to the medical staff after an accident occurred during sports and non-sports activities, and in particular all those activities that require the use of a protective helmet.

The aforementioned technical purpose and objects are substantially fulfilled by a sensorized garment that comprises the technical features as disclosed in one or more of the accompanying claims.

### Advantages of the Invention

One embodiment provides a sensorized garment according to claim 1, that can provide the medical staff, especially in first aid situations, with reliable information about the traumas suffered by the person involved in the accident that wears the sensorized garment.

One embodiment provides a sensorized system according to claim 8, that can provide a virtual map for display to the medical staff and represents the amount of traumas that has been suffered by the individual during the accident.

### BIEF DESCRIPTION OF THE DRAWINGS

The characteristics and advantages of the present invention will result from the following detailed description of a possible practical embodiment, illustrated as a nonlimiting example in the set of drawings, in which:

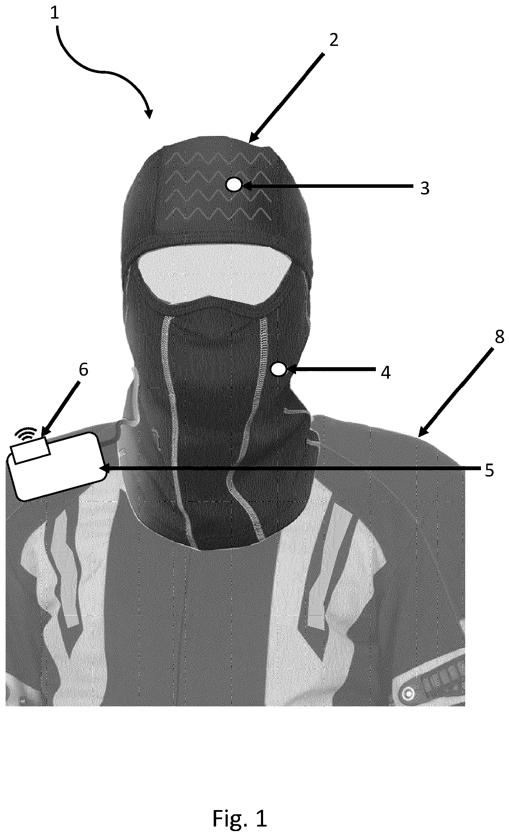

- Figure 1 shows a sensorized garment of the present invention;
- Figure 2 shows a view of a virtual map generated by means of the sensorized garment of Figure 1,
- Figure 3 shows one example of application of the sensorized garment of Figure 1 and of virtual map of Figure 2 generated thereby,
- Figure 4 shows a side view of a sensorized garment according to a further embodiment of the present invention,
- Figure 5 shows the sensorized garment of Figure 4 with additional construction details,
- Figure 6 shows a rear view of the of the sensorized garment of Figure 4,
- Figure 7 shows a front view of a sensorized garment according to a further embodiment of the present invention,
- Figure 8 shows a rear view of the sensorized garment of Figure 7.

### DETAILED DESCRIPTION

Even when this is not expressly stated, the individual features as described with reference to the particular embodiments shall be intended as auxiliary to and/or interchangeable with other features described with reference to other exemplary embodiments.

The sensorized garment of the annexed figures shall be deemed to be schematically illustrated, not necessarily drawn to scale, and not necessarily representing the actual proportions of its parts.

The present invention relates to a sensorized garment 1 comprising an under helmet hood 2 made of a textile material that can be worn to cover at least the head and neck of an individual, preferably also the shoulders.

As used herein, the term under helmet hood 2 is intended to designate a textile hood having the form of a balaclava, i.e. entirely covering the head and neck, with an opening that leaves the eyes, and optionally also the nose and mouth, exposed, which is used in sports that require the use of a safety helmet. The under helmet hood 2 is adapted to be worn under a protective helmet.

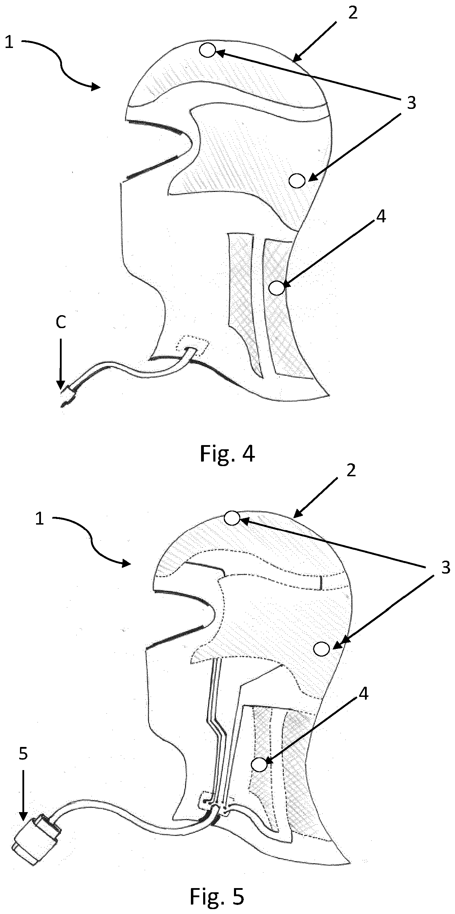

Furthermore, reference is made herein to textile sensorized elements, i.e. having electrical connections incorporated in the textile materials to provide signal communication with the various electronic components, also incorporated in the textile element (Figure 5). The electronic elements incorporated in the textile element are intended, for example, as the sensors described hereinafter. These sensors and their mutual electrical connections are stably incorporated in the textile element with techniques known in the art such as stitching or molding.

The sensorized garment 1 comprises at least one textile pressure sensor 3 and at least one textile deformation sensor 4 incorporated in the under helmet hood 2. Both the pressure sensor 3 and the deformation sensor 4 are configured to contact the body surface of said individual and are therefore positioned on the under helmet hood 2 to directly interface with the body surface of the individual.

Each pressure sensor 3 is configured to sense a pressure value and generate a first signal in response to the pressure value it has sensed.

Each deformation sensor 4 is configured to sense a deformation value and generate a second signal in response to the deformation value it has sensed.

The sensorized garment 1 comprises a control unit 5 in signal communication with each pressure sensor 3 and each deformation sensor 4 to receive the first signal and the second signal respectively. The control unit 5 is configured to generate an alarm signal when the value of at least one of the first signal and the second signal exceeds a respective preset threshold value. Preferably, the threshold value of the first signal corresponds to the intensity of the compressive force that acts on the body surface beyond which the wearer of the garment 1 would be injured. Likewise, the threshold value of the second signal corresponds to the maximum deformation value for the under helmet hood 2, and hence the maximum elongation of the body surface beyond which the wearer of the sensorized garment 1 would be injured.

Preferably, the control unit 5 is adapted to be removably engaged to the under helmet hood 2. Advantageously, the control unit 5 may be removed and may be used with different under helmet hoods 2.

Advantageously, during operation, the sensorized garment is able to detect compressions and extensions of the wearer's head that might cause traumas and injuries during an impact.

According to the preferred embodiment of the invention, as shown in the Figures 4 to 6, each pressure sensor 3 is positioned in a portion of the under helmet hood 2 which is designed to contact one or more regions selected from forehead, scalp and temples of a wearer of the sensorized garment 1.

According to the preferred embodiment, the pressure sensor 3 is positioned in a portion of the under helmet hood 2 that is designed to contact a region selected from nose, jaws, cheeks and cheekbones of a wearer of the sensorized garment 1. By this arrangement the pressure sensor 3 may be advantageously interfaced, during operation, with one of the above listed portions of the body surface of the individual. Thus, when an individual wears the sensorized garment 1, namely the under helmet hood 2 under a helmet, the above listed regions of the wearer's head are most exposed to impact and therefore to compression forces during impacts.

Preferably, the pressure sensor 3 is a piezoelectric sensor.

In the preferred embodiment of the invention, each deformation sensor 4 is positioned in a portion of the under helmet hood 2 that is designed to contact a region of the neck of an individual when he/she wears the sensorized garment 1, and hence the under helmet hood 2. Once again, due to their position, the deformation sensors 4 may sense deformations, namely extensions in the region of the wearer's head that is more exposed to extension during impacts, i.e. the neck.

Preferably, the deformation sensor 4 is a strain gage.

According to a preferred embodiment of the invention, the sensorized garment 1 comprises a plurality of pressure sensors 3 of the aforementioned type, which are configured to generate a plurality of first signals.

Still preferably, the sensorized garment 1 comprises a plurality of deformation sensors 4 of the aforementioned type, which are configured to generate a plurality of second signals.

According to a non-claimed embodiment, not shown, the sensorized garment 1 comprises one or more acceleration sensors, preferably an accelerometer. Each acceleration sensor is incorporated into the under helmet hood 2, is configured to detect speed variations to generate a third signal in response to the detected value of speed.

Each acceleration sensor is in signal communication with the control unit 5. Preferably, the control unit 5 is configured to receive the third signal and generate the alarm signal when the value of the third signal exceeds a preset threshold value. Thus, the control unit 5 is able to detect the imminent impact in advance, and determine its severity according to the detected acceleration. Furthermore, the control unit 5 is preferably configured to detect speed variations also after the main impact, to determine whether the individual moved after the accident. This information may be crucial during first aid.

According to a preferred embodiment of the invention, the sensorized garment 1 comprises a transceiver unit 6 associated with the control unit 5 and connected to a mobile network to transmit the alarm signal, the first signal, the second signal and, if any, also the third signal, to an external processing unit 7 connected to the mobile network.

As used herein, the term mobile network is intended to designate a GSM-based telephone network in its various developments such as 3G, 4G, LTE and 5G as well as other data transmission networks such as Wi-Fi, based on protocols known to a skilled person.

Advantageously, the sensorized garment 1 can provide the data collected during an accident to the external processing unit 7, which can process the data to generate a report concerning the dynamics of the impact suffered by the wearer of the sensorized garment 1. It shall be noted that such information reaches the first aid medical staff, who will quickly understand the dynamics of the impact to consider the best strategy for first aid and treatment of the individual involved in the accident.

According to a further preferred embodiment, the transceiver means 6 are configured to connect to a smartphone, a tablet, a PC or a similar external electronic device to transmit the alarm signal, the first signal, the second signal and, if any, also the third signal. These external electronic devices include the external processing unit 7, or transmit the data received from the control unit 5 to a remote external processing unit 7, for example using the aforementioned data transmission networks.

In one aspect, the control unit 5 is configured to generate geolocation data G indicative of the geographic location of the sensorized garment and to send the geolocation data G to the external processing unit 7. The geolocation data G are generated by connection to the mobile network, i.e. the 3G, 4G, LTE and more preferably 5G network.

Alternatively, the geolocation data G may be generated using GPS.

Advantageously, the external processing unit 7 may identify the exact geographic location of the sensorized garment 1, and hence the individual involved in an accident, to thereby be able to provide such geographical coordinates to the first aid mobile units that can quickly identify and reach the wearer of the sensorized garment 1.

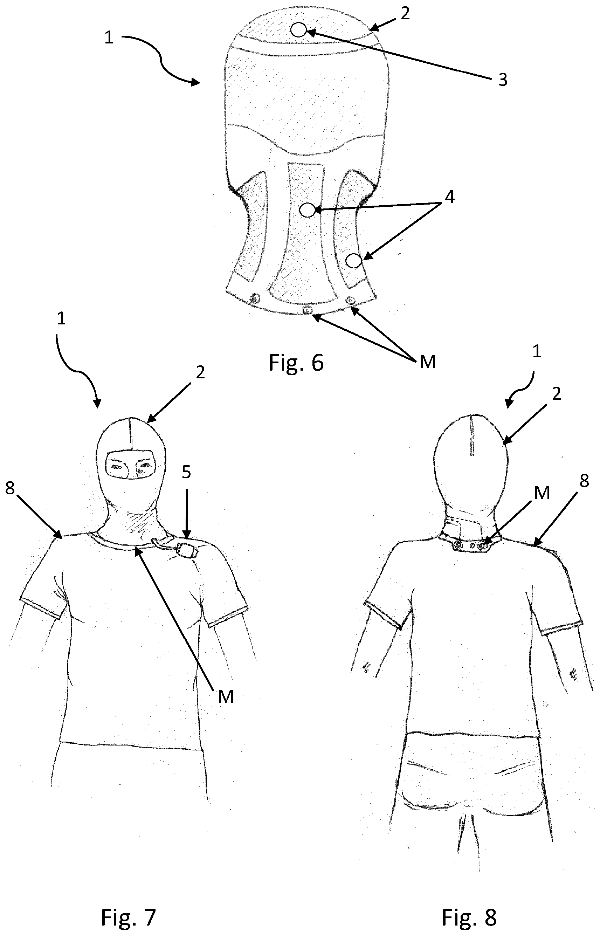

According to a preferred embodiment of the invention, as shown in Figures 7 and 8, the sensorized garment comprises a t-shirt 8 made of textile material and adapted to be worn by an individual. The t-shirt 8 and the under helmet hood 2 are connected at least at their respective neck portions by fastening means M, preferably one or more of seams, zippers, snap fasteners, Velcro or press studs. Advantageously, the connection between these two textile elements, i.e. the under helmet hood 2 and the t-shirt 8, will afford improved operation of the deformation sensors 4, especially in elongation detection. This is because the t-shirt 8 can always keep the neck portion of the under helmet hood 2 in position, thereby preventing any slipping that might occur when the hood 2 is stretched and that might alter extension detection or even cause slipping of the under helmet hood 2 off the wearer's head.

Preferably, at least one pressure sensor 3, at least one deformation sensor 4 and optionally at least one acceleration sensor, are also incorporated in the t-shirt 8 to provide the same measurements as described above.

According to a preferred embodiment of the invention, the control unit 5 transmits the first signals, the second signals and optionally the third signals to the external processing unit 7 for a preset duration of time in response to the first event in which the threshold value of at least one of the aforementioned signals is detected to be exceeded, i.e. Upon generation of an alarm signal.

According to a preferred embodiment, the sensorized garment 1 comprises a memory unit (not shown). Preferably, the control unit 5 stores all the first, second and optionally third signals generated during a preset interval of time after the threshold value of at least one of these signals has been exceeded, i.e. after the generation of an alarm signal, in the memory unit.

Advantageously, the sensorized garment 1 tracks all compression, extension and optionally acceleration events occurring during a preset interval of time upon generation of an alarm signal. In other words, the compression, extension and acceleration values are detected and sent to an external unit over a period of time following the accident. This will provide a complete picture of the traumatic events suffered by the wearer of the sensorized garment. In particular, the detection of speed variations after the main impact by means of one or more acceleration sensors, provides information about any movements of the person after the accident. This information is particularly useful for the first aid medical staff to ascertain the actual health conditions of the individual after the accident.

Preferably, the sensorized under helmet hood is equipped with a battery unit for power supply to all of its components.

Advantageously, the sensorized garment 1 is compact, comfortable, hygienic, breathable and easy to wash.

Advantageously, the control unit is actuated as soon as the garment is fastened and remains silent until the time in which an alarm signal is generated and sent to the external processing unit 7. This involves considerable energy savings.

The present invention also relates to a sensorized system and has the purpose to detect and map the impacts that the wearer of the inventive sensorized garment suffers.

For this purpose, the sensorized system comprises the sensorized garment 1 having a plurality of pressure sensors 3 of the aforementioned type, which are configured to generate a plurality of first signals. Also, the sensorized garment 1 comprises a plurality of deformation sensors 4 of the aforementioned type, which are configured to generate a plurality of second signals. Optionally, the sensorized garment 1 comprises a plurality of acceleration sensors of the aforementioned type, which are configured to generate a plurality of third signals.

The sensorized system also comprises an external processing unit 7 in signal communication with the sensorized garment 1.

The control unit 5 is configured to transmit the alarm signal, the plurality of first signals and the plurality of second signals and optionally also the plurality of third signals to the external processing unit 7.

The external processing unit 7 is configured to process the plurality of first signals to generate first map data representative of a first map of the pressure values detected by the plurality of pressure sensors 3 on the surface of said under helmet hood 2.

The external processing unit 7 is configured to process said plurality of second signals to generate second map data representative of a second map of the deformation values detected by the plurality of deformation sensors 4 on the surface of the under helmet hood 2.

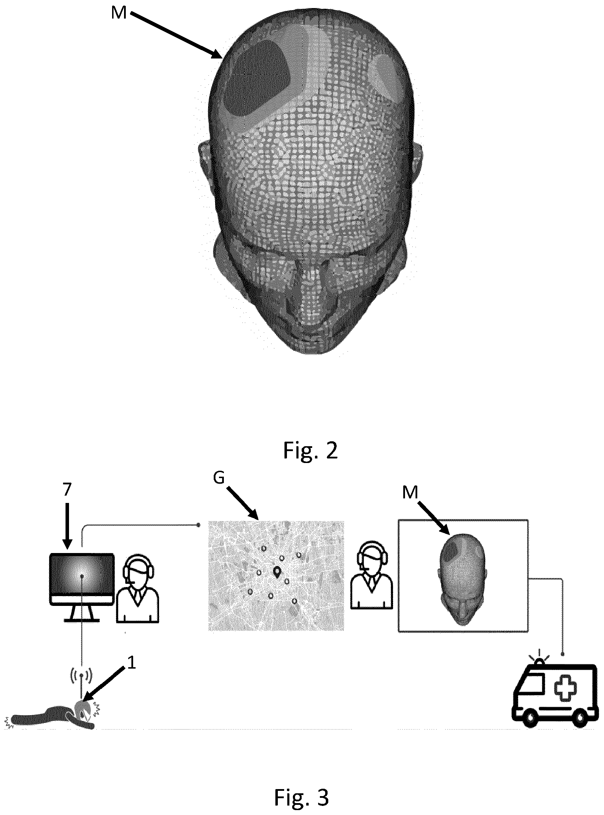

The external processing unit 7 is configured to combine the first map data and the second map data to generate a virtual image map M representative of the detected deformation and compression values and their distribution on the surface of the under First data helmet hood 2. Preferably, the virtual map image M is a three-dimensional map or composed of one or more two-dimensional images, which provide a visual indication of the areas of the sensorized garment 1 that have suffered the impact or impacts, i.e. the compressions and/or deformations. More preferably, the virtual image map M provides a visual indication of the distribution and value of the compressions and deformations on the surface of the sensorized garment 1, and particularly on the surface of the under helmet hood 2 and the t-shirt 8 (if any). Such visual indication is preferably obtained with the definition of colored areas. The extent of each colored area indicates the region of the head affected by compressions and/or deformations, whereas the intensity of the color is related to the intensity of the compression and/or deformation values that have been detected. Advantageously, the virtual image map M as shown in Figures 2 and 3 may be provided to the first aid medical staff and is thus useful in diagnosing the individual involved in the accident.

## Claims

1. A sensorized garment (1) comprising:
- an under helmet hood (2) made of a textile material, which can be worn to cover at least the head of an individual;
- at least one textile pressure sensor (3) and at least one textile deformation sensor (4) incorporated in said under helmet hood (2) and configured to contact the body surface of said individual, each pressure sensor (3) being configured to detect a pressure value to thereby generate a first signal according to said detected pressure value, each deformation sensor (4) being configured to detect a deformation value to thereby generate a second signal according to said detected deformation value;
- a control unit (5) in signal communication with said at least one textile pressure sensor (3) and with said at least one textile deformation sensor (4) for receiving said first signal and said second signal respectively, said control unit (5) being configured to generate an alarm signal when the value of at least one of said first signal and said second signal exceeds a respective preset threshold value;
- each textile pressure sensor (3) is placed in a portion of said under helmet hood (2) which is designed to contact one or more areas selected from forehead, scalp, temples, nose, jaws, cheeks and cheekbones of said individual;
**characterized in that**
- the under helmet hood (2) can be worn to cover also the neck of said individual, and each textile deformation sensor (4) is placed in a portion of said under helmet hood (2) which is designed to contact an area of the neck of said individual.

2. A sensorized garment (1) as claimed in claim 1, comprising a transceiver unit (6) associated with said control unit (5) and connected to a mobile network to transmit said alarm signal, said first signal and said second signal to an external processing unit (7) connected to said mobile network.

3. A sensorized garment (1) as claimed in claim 1, wherein said control unit (5) is configured to generate geolocation data (G) indicative of the geographic location of said sensorized garment (1) and to send said geolocation data (G) to said external processing unit (7).

4. A sensorized garment (1) as claimed in any of the preceding claims, wherein said textile pressure sensor (3) is a piezoelectric sensor.

5. A sensorized garment (1) as claimed in any of the preceding claims, wherein said textile deformation sensor (4) is a strain gage.

6. A sensorized garment (1) as claimed in any of the preceding claims, comprising a t-shirt (8) made of textile material which can be worn by said individual, said t-shirt (8) and said under helmet hood (2) being connected to each other at least at their respective neck portions.

7. A sensorized garment (1) as claimed in claim 6, wherein said at least one textile pressure sensor (3) and at least one textile deformation sensor (4) are also incorporated in said t-shirt (8).

8. A sensorized system for detecting and mapping impacts, comprising:
- a sensorized garment (1) as claimed in any of the preceding claims 1 to 7;
- an external processing unit (7) in signal communication with said sensorized garment (1);
said system being **characterized in that:**
- said at least one textile pressure sensor (3) comprises a plurality of textile pressure sensors (3) configured to generate a plurality of first signals;
- said at least one textile deformation sensor (4) comprises a plurality of deformation sensors (4) configured to generate a plurality of second signals;
- said control unit (5) is configured to transmit said alarm signal, said plurality of first signals and said plurality of second signals to said external processing unit (7);
- said external processing unit (7) is configured to process said plurality of first signals to generate first map data representative of a first map of the pressure values detected by said plurality of pressure sensors (3) on the surface of said under helmet hood (2);
- said external processing unit (7) is configured to process said plurality of second signals to generate second map data representative of a second map of the deformation values detected by said plurality of deformation sensors (4) on the surface of said under helmet hood (2);
- said external processing unit (7) is configured to combine said first map data and said second map data to generate the image of a virtual map (M) representative of the detected deformation and compression values and their distribution on the surface of said under helmet hood (2).

## Patentansprüche

1. Mit Sensoren versehenes Kleidungsstück (1), umfassend:
- eine Unterhelmhaube (2) aus einem textilen Material, die getragen werden kann, um mindestens den Kopf einer Person zu bedecken;
- mindestens einen Textildrucksensor (3) und mindestens einen Textilverformungssensor (4), die in die Unterhelmhaube (2) eingearbeitet und so konfiguriert sind, dass sie die Körperoberfläche der Person berühren, wobei jeder Drucksensor (3) so konfiguriert ist, dass er einen Druckwert erfasst, um dadurch ein erstes Signal entsprechend dem erfassten Druckwert zu erzeugen, und jeder Verformungssensor (4) so konfiguriert ist, dass er einen Verformungswert erfasst, um dadurch ein zweites Signal entsprechend dem erfassten Verformungswert zu erzeugen;
- eine Steuereinheit (5) in Signalkommunikation mit dem mindestens einen Textildrucksensor (3) und mit dem mindestens einen Textilverformungssensor (4) zum Empfangen des ersten Signals bzw. des zweiten Signals, wobei die Steuereinheit (5) so konfiguriert ist, dass sie ein Alarmsignal erzeugt, wenn der Wert des ersten Signals und/oder des zweiten Signals einen jeweiligen voreingestellten Schwellenwert überschreitet;
- jeder Textildrucksensor (3) ist in einem Teil der Unterhelmhaube (2) angeordnet, der dazu bestimmt ist, einen oder mehrere Bereiche zu berühren, die aus Stirn, Kopfhaut, Schläfen, Nase, Kiefer, Wangen und Wangenknochen der Person ausgewählt sind;
**dadurch gekennzeichnet, dass**
- die Unterhelmhaube (2) so getragen werden kann, dass sie auch den Hals der Person bedeckt, und jeder Textilverformungssensor (4) in einem Teil der Unterhelmhaube (2) angeordnet ist, der dazu bestimmt ist, einen Bereich des Halses der Person zu berühren.

2. Mit Sensoren versehenes Kleidungsstück (1) nach Anspruch 1, umfassend eine Sende-Empfangs-Einheit (6), die mit der Steuereinheit (5) verbunden ist und an ein Mobilfunknetz angeschlossen ist, um das Alarmsignal, das erste Signal und das zweite Signal an eine externe Verarbeitungseinheit (7) zu übertragen, die an das Mobilfunknetz angeschlossen ist.

3. Mit Sensoren versehenes Kleidungsstück (1) nach Anspruch 1, wobei die Steuereinheit (5) so konfiguriert ist, dass sie Geolokalisierungsdaten (G) erzeugt, die den geografischen Standort des mit Sensoren versehenen Kleidungsstücks (1) anzeigen, und die Geolokalisierungsdaten (G) an die externe Verarbeitungseinheit (7) sendet.

4. Mit Sensoren versehenes Kleidungsstück (1) nach einem der vorstehenden Ansprüche, wobei der Textildrucksensor (3) ein piezoelektrischer Sensor ist.

5. Mit Sensoren versehenes Kleidungsstück (1) nach einem der vorstehenden Ansprüche, wobei der Textilverformungssensor (4) ein Dehnungsmessstreifen ist.

6. Mit Sensoren versehenes Kleidungsstück (1) nach einem der vorstehenden Ansprüche, umfassend ein T-Shirt (8) aus textilem Material, das von der Person getragen werden kann, wobei das T-Shirt (8) und die Unterhelmhaube (2) mindestens an ihren jeweiligen Halsabschnitten miteinander verbunden sind.

7. Mit Sensoren versehenes Kleidungsstück (1) nach Anspruch 6, wobei der mindestens eine Textildrucksensor (3) und der mindestens eine Textilverformungssensor (4) auch in das T-Shirt (8) eingearbeitet sind.

8. Mit Sensoren versehenes System zum Erkennen und Kartieren von Aufschlägen, umfassend:
- ein mit Sensoren versehenes Kleidungsstück (1) nach einem der vorstehenden Ansprüche 1 bis 7;
- eine externe Verarbeitungseinheit (7), die in Signalkommunikation mit dem mit Sensoren versehenen Kleidungsstück (1) steht;
wobei das System **dadurch gekennzeichnet ist, dass:**
- der mindestens eine Textildrucksensor (3) eine Mehrzahl von Textildrucksensoren (3) umfasst, die so konfiguriert sind, dass sie eine Mehrzahl von ersten Signalen erzeugen;
- der mindestens eine Textilverformungssensor (4) eine Mehrzahl von Verformungssensoren (4) umfasst, die so konfiguriert sind, dass sie eine Mehrzahl von zweiten Signalen erzeugen;
- die Steuereinheit (5) so konfiguriert ist, dass sie das Alarmsignal, die Mehrzahl der ersten Signale und die Mehrzahl der zweiten Signale an die externe Verarbeitungseinheit (7) überträgt;
- die externe Verarbeitungseinheit (7) so konfiguriert ist, dass sie die Mehrzahl der ersten Signale verarbeitet, um erste Kartendaten zu erzeugen, die repräsentativ für eine erste Karte der von der Mehrzahl von Drucksensoren (3) auf der Oberfläche der Unterhelmhaube (2) erfassten Druckwerte sind;
- die externe Verarbeitungseinheit (7) so konfiguriert ist, dass sie die Mehrzahl der zweiten Signale verarbeitet, um zweite Kartendaten zu erzeugen, die repräsentativ für eine zweite Karte der von der Mehrzahl von Verformungssensoren (4) auf der Oberfläche der Unterhelmhaube (2) erfassten Verformungswerte sind;
- die externe Verarbeitungseinheit (7) so konfiguriert ist, dass sie die ersten Kartendaten und die zweiten Kartendaten kombiniert, um das Bild einer virtuellen Karte (M) zu erzeugen, die repräsentativ für die erfassten Verformungs- und Druckwerte und deren Verteilung auf der Oberfläche der Unterhelmhaube (2) ist.

## Revendications

1. Vêtement muni de capteurs (1) comprenant :
- une capuche de sous-casque (2) faite d'un matériau textile, qui peut être portée pour couvrir au moins la tête d'un individu ;
- au moins un capteur de pression textile (3) et au moins un capteur de déformation textile (4) incorporés dans ladite capuche de sous-casque (2) et configurés pour entrer en contact avec la surface du corps dudit individu, chaque capteur de pression (3) étant configuré pour détecter une valeur de pression pour ainsi générer un premier signal selon ladite valeur de pression détectée ; chaque capteur de déformation (4) étant configuré pour détecter une valeur de déformation pour ainsi générer un second signal selon ladite valeur de déformation détectée ;
- une unité de commande (5) en communication par signaux avec ledit au moins un capteur de pression textile (3) et avec ledit au moins un capteur de déformation textile (4) pour recevoir ledit premier signal et ledit second signal respectivement, ladite unité de commande (5) étant configurée pour générer un signal d'alarme lorsque la valeur d'au moins un dudit premier signal et dudit second signal dépasse une valeur de seuil prédéfinie respective ;
- chaque capteur de pression textile (3) est placé dans une partie de ladite capuche de sous-casque (2) conçue pour entrer en contact avec une ou plusieurs zones choisies parmi le front, le cuir chevelu, les tempes, le nez, les mâchoires, les joues et les pommettes dudit individu ;
**caractérisé en ce que**
- la capuche de sous-casque (2) peut être portée pour couvrir également le cou dudit individu, et chaque capteur de déformation textile (4) est placé dans une partie de ladite capuche de sous-casque (2) conçue pour entrer en contact avec une zone du cou dudit individu.

2. Vêtement muni de capteurs (1) selon la revendication 1, comprenant une unité d'émission-réception (6) associée à ladite unité de commande (5) et connectée à un réseau mobile pour transmettre ledit signal d'alarme, ledit premier signal et ledit second signal à une unité de traitement externe (7) connectée au dit réseau mobile.

3. Vêtement muni de capteurs (1) selon la revendication 1, dans lequel ladite unité de commande (5) est configurée pour générer des données de géolocalisation (G) indiquant l'emplacement géographique dudit vêtement muni de capteurs (1) et pour envoyer lesdites données de géolocalisation (G) à ladite unité de traitement externe (7).

4. Vêtement muni de capteurs (1) selon l'une quelconque des revendications précédentes, dans lequel ledit capteur de pression textile (3) est un capteur piézoélectrique.

5. Vêtement muni de capteurs (1) selon l'une quelconque des revendications précédentes, dans lequel ledit capteur de déformation textile (4) est une jauge de contrainte.

6. Vêtement muni de capteurs (1) selon l'une quelconque des revendications précédentes, comprenant un t-shirt (8) en matière textile qui peut être porté par ledit individu, ledit t-shirt (8) et ladite capuche de sous-casque (2) étant reliés l'un à l'autre au moins au niveau de leurs parties de cou respectives.

7. Vêtement muni de capteurs (1) selon la revendication 6, dans lequel ledit au moins un capteur de pression textile (3) et au moins un capteur de déformation textile (4) sont également incorporés dans ledit t-shirt (8).

8. Système de capteurs permettant de détecter et cartographier des impacts, comprenant :
- un vêtement muni de capteurs (1) selon l'une quelconque des revendications 1 à 7 précédentes ;
- une unité de traitement externe (7) en communication par signal avec ledit vêtement muni de capteurs (1) ;
ledit système étant **caractérisé en ce que** :
- ledit au moins un capteur de pression textile (3) comprend une pluralité de capteurs de pression textile (3) configurés pour générer une pluralité de premiers signaux ;
- ledit au moins un capteur de déformation textile (4) comprend une pluralité de capteurs de déformation (4) configurés pour générer une pluralité de seconds signaux ;
- ladite unité de commande (5) est configurée pour transmettre ledit signal d'alarme, ladite pluralité de premiers signaux et ladite pluralité de seconds signaux à ladite unité de traitement externe (7) ;
- ladite unité de traitement externe (7) est configurée pour traiter ladite pluralité de premiers signaux pour générer des premières données de carte représentatives d'une première carte des valeurs de pression détectées par ladite pluralité de capteurs de pression (3) sur la surface de ladite capuche de sous-casque (2) ;
- ladite unité de traitement externe (7) est configurée pour traiter ladite pluralité de seconds signaux pour générer des secondes données de carte représentatives d'une seconde carte des valeurs de déformation détectées par ladite pluralité de capteurs de déformation (4) sur la surface de ladite capuche de sous-casque (2) ;
- ladite unité de traitement externe (7) est configurée pour combiner lesdites premières données de carte et lesdites secondes données de carte pour générer l'image d'une carte virtuelle (M) représentative des valeurs de déformation et de compression détectées et de leur distribution sur la surface de ladite capuche de sous-casque (2).
